# FASCICULE DE BREVET EUROPEEN

(11) **EP 1 405 625 B1**
(45) Date de publication et mention de la délivrance du brevet: **24.03.2010**
(21) Numéro de dépôt: 03020174.3
(22) Date de dépôt: 05.09.2003
(51) Int. Cl.: A61Q 1/04, A61K 8/85, A61K 8/891

(54) **Composition cosmétique comprenant des huiles, un agent rhéologique et une phase particulaire**
Kosmetische Zusammensetzung enthaltend Öle, ein rheologisches Mittel und eine Teilchenphase
Cosmetic composition comprising oils, a rheological agent and a particulate phase

(30) Priorité: 06.09.2002 FR 0211095
(43) Date de publication de la demande: 07.04.2004
(60) Demande divisionnaire: 06300700.9
(73) Titulaire: L'Oréal, 75008 Paris (FR)
(72) Inventeur: Blin, Xavier, 75015 Paris (FR); Ferrari, Véronique, 94700 Maisons-Alfort (FR)
(74) Mandataire: Le Coupanec, Pascale A.M.P.

(56) Documents cités:
- EP-A- 0 819 419
- EP-A- 1 249 223
- EP-A- 1 249 226
- FR-A- 2 771 628
- DATABASE WPI Week 198644 Derwent Publications Ltd., London, GB; AN 1986-287936 XP002246654 & JP 61 210018 A (SHISEIDO), 18 septembre 1986 (1986-09-18)

## Description

La présente invention se rapporte à une composition cosmétique comprenant, dans un milieu physiologiquement acceptable, une huile siliconée phénylée de haute viscosité, une huile hydrocarbonée non volatile, un agent rhéologique et une phase particulaire. Cette composition est notamment une composition de maquillage ou de soin de la peau, aussi bien du visage que du corps humain, y compris du cuir chevelu, des lèvres ou des phanères des êtres humains, comme les cheveux, les cils, les sourcils ou les ongles. Elle possède des propriétés cosmétiques remarquables, en particulier de tenue, et confère au maquillage ou au soin des propriétés de brillance et/ou de confort.

La composition de l'invention peut en particulier se présenter sous forme d'un produit de maquillage des matières kératiniques (peau, lèvres, phanères) ayant éventuellement des propriétés de soin et/ou de traitement non thérapeutique. Elle constitue notamment un rouge à lèvres ou un brillant à lèvres, un fond de teint, une poudre libre ou compactée, un fard à joues ou à paupières, une base de maquillage, un produit anticernes, un produit pour tatouage, un mascara, un eye-liner, un vernis à ongles, un produit de bronzage artificiel de la peau, un produit de coloration ou de soin des cheveux.

L'utilisation de composés siliconés dans des compositions cosmétiques, notamment de maquillage, est connue des formulateurs. Il a été décrit une composition comprenant l'association d'une gomme de silicone et d'une huile siliconée, présentant de bonnes propriétés de tenue, d'étalement et de confort.
En particulier, cette association confère aux compositions cosmétiques d'excellentes propriétés sensorielles, notamment un toucher non gras, des propriétés d'étalement et de glissant et permettent d'obtenir sur la peau un film particulièrement homogène.

Il est également connu d'utiliser ces composés siliconés en vue d'augmenter la tenue des compositions cosmétiques, en particulier de maquillage. Les problèmes de mauvaise tenue se caractérisent par une modification de la couleur (virage, palissement) généralement par suite d'une interaction avec le sébum et/ou la sueur sécrétés par la peau dans le cas de fond de teint et de fard ou d'une interaction avec la salive dans le cas des rouges à lèvres. Ceci oblige l'utilisateur à se remaquiller très souvent, ce qui peut constituer une perte de temps.

Cependant, ces composés siliconés peuvent parfois poser des problèmes de formulation, et notamment des difficultés de dispersion des pigments dans les compositions comprenant des milieux siliconés, ce qui entraîne un mauvais développement de la teinte de la composition ainsi qu'un aspect granuleux, qui éloigne le consommateur de ce type de produit, et qui n'est pas favorable à l'obtention d'une composition brillante.

Or il existe de nombreuses compositions cosmétiques pour lesquelles les propriétés de brillance du film déposé, après application sur les matières kératiniques (peau, lèvres, phanères) sont très importantes. On peut citer par exemple les rouges à lèvres, les fards à paupières, les vernis à ongles ou encore certains produits capillaires.

Le document FR 2 771 628 décrit l'utilisation d'une gomme de siliconée solubilisée dans une huile phényltriméthicone dans une composition cosmétique. Cette gomme présente l'avantage d'être soluble avec les huiles couramment utilisées en cosmétique.

Le document EP 1 112 734 décrit une composition sans transfert contenant une huile hydrocarbonée et une huile siliconée, lesquelles ne sont pas solubles l'une dans l'autre. La composition contient en outre un solvant volatile.

Le document JP 61-210 018 concerne une composition contenant une huile de viscosité comprise entre 1000 et 500 000 cps et un agent rhéologique.

Les documents EP 1 249 223 et EP 1 249 226 décrivent l'association d'une huile siliconée volatile, d'une huile hydrocarbonée, d'un pigment et d'un agent rhéologique dans une première composition, sur laquelle on applique une deuxième composition contenant une huile siliconée de haute viscosité.

Pour l'amélioration de la brillance, il est connu des formulateurs d'utiliser des huiles ayant une viscosité et un indice de réfraction élevés telles que des polymères huileux comme les polybutènes ou certaines huiles végétales (huile de ricin par exemple). Cependant, ces composés ne permettent pas l'obtention d'un film de composition dont la tenue, en particulier de la brillance, persiste au cours de la journée.

Le demandeur a constaté de façon surprenante que l'utilisation de l'association d'une huile siliconée phénylée de haute viscosité et d'une huile hydrocarbonée non volatile solubles ou dispersibles l'une dans l'autre permet l'obtention d'une composition présentant de bonnes propriétés cosmétiques et sensorielles, notamment de tenue dans le temps, de brillance et de confort et qui est non irritante pour les matières kératiniques.

La brillance moyenne est mesurée à l'aide d'un brillancemètre, de manière conventionnelle, par la méthode suivante. Sur une carte de contraste de marque LENETA et de référence FORM 1A PENOPAC, on étale une couche de 50 µm d'épaisseur de la composition dont on cherche à évaluer la brillance moyenne, à l'aide d'un étaleur automatique. La couche recouvre au moins le fond blanc de la carte. On procède immédiatement à la mesure de la brillance à 20° sur le fond blanc à l'aide d'un brillancemètre de marque BYK GARDNER et de référence microTRl-GLOSS. La brillance de la composition peut être également mesurée une heure après le dépôt de la composition.

La brillance moyenne de la composition mesurée au dépôt est de préférence supérieure à 120, 130, 140, 150, et mieux 160 sur 200.

La brillance moyenne de la composition mesurée une heure après le dépôt est de préférence supérieure à 120, et mieux 130 sur 200.

La tenue après épreuve de la composition selon l'invention peut être évaluée de la façon suivante sur un groupe d'au moins 10 personnes. On mesure, sur chaque personne, à l'aide d'une caméra, le coefficient de diffusion réduit de la lumière incidente qui éclaire les lèvres nues sur laquelle on désire appliquer la composition cosmétique, puis on calcule la moyenne des mesures. Les conditions de mesure sont décrites dans la demande FR0207108. Chaque personne applique ensuite la composition cosmétique sur les lèvres nues, puis applique un mouchoir en papier dessus sans frotter, par simple contact. On mesure comme précédemment le coefficient de diffusion réduit des lèvres ainsi maquillées pour chaque personne on fait la moyenne. Chaque personne participant à l'évaluation de la tenue boit ensuite une boisson chaude puis un verre d'eau et mange quatre bouchée d'un sandwich et une demie pomme. A la fin de ce repas, on mesure comme précédemment le coefficient de diffusion réduit des lèvres maquillées pour chaque personne et on calcule la moyenne.
La tenue après épreuve est exprimée comme le pourcentage de a) la différence entre le coefficient de diffusion réduit moyen des lèvres maquillées, après le repas, et le coefficient de diffusion réduit moyen des lèvres nues par rapport à b) la différence entre le coefficient de diffusion réduit moyen des lèvres maquillées, avant le repas, et le coefficient de diffusion réduit moyen des lèvres nues.

La tenue après épreuve de la composition selon l'invention est supérieure à 40 sur 100, de préférence supérieure à 50 sur 100.

La présente invention a pour objet une composition cosmétique comprenant, dans un milieu physiologiquement acceptable, a) au moins une huile siliconée phénylée de haute viscosité présentant une viscosité à 25 °C allant de 5.10⁻⁴ à 1.10⁻² m²/s (500 à 10 000 cSt), b) au moins une huile hydrocarbonée non volatile ayant une masse moléculaire supérieure à 600 g/mol choisie parmi les polyesters hydroxylés, lesdits polyesters hydroxylés étant des polyesters de monoalcool gras et d'acide polycarboxylique hydroxylé, l'huile siliconée phénylée de haute viscosité étant choisie parmi les huiles de formule (A) suivante : 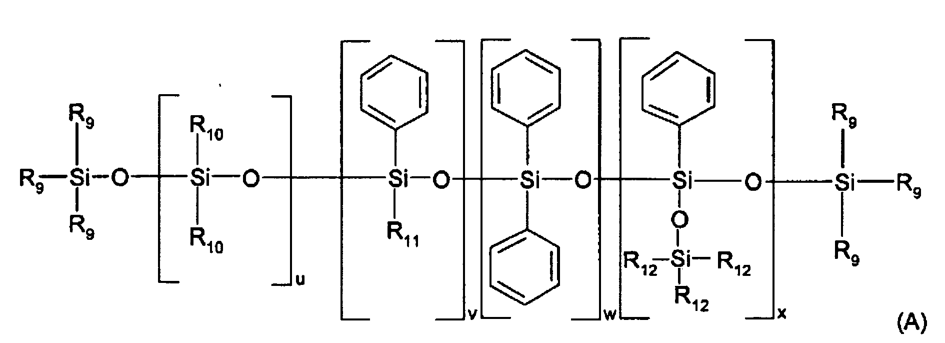 dans laquelle :
- R₉ et R₁₂ sont chacun indépendamment un radical alkyle en C₁-C₃₀, un radical aryle ou un radical aralkyle,
- R₁₀ et R₁₁ sont chacun indépendamment un radical alkyle en C₁-C₃₀ ou un radical aralkyle,
- u, v, w et x sont chacun indépendamment des nombres entiers allant de 0 à 900, sous réserve que la somme v+w+x soit différente de 0 et que la somme u+v+w+x va de 1 à 900, en particulier, u+v+w+x va de 1 à 800 et c) une phase particulaire, caractérisée en ce qu'elle comprend moins de 5% d'une huile volatile.

La présente invention a également pour objet l'utilisation de l'association d'au moins une huile siliconée phénylée de haute viscosité supérieure ou égale à 5.10⁻⁴ m²/s (500 cSt), d'au moins une huile hydrocarbonée non volatile de masse moléculaire supérieure à 600 g/mol soluble ou dispersible dans ladite huile siliconée, l'huile hydrocarbonée non volatile étant choisie parmi les polyesters hydroxylés, lesdits polyesters hydroxylés étant des polyesters de monoalcool gras et d'acide polycarboxylique hydroxylé, dans une composition comprenant un milieu physiologiquement acceptable et une phase particulaire et comprenant moins de 5 % d'une huile volatile, ladite composition présentant des propriétés de tenue et de brillance.

L'invention a encore pour objet un procédé cosmétique pour conférer à un film de composition cosmétique comprenant une phase particulaire et moins de 5 % d'une huile volatile des propriétés de tenue et de brillance, consistant à introduire dans ladite composition une quantité efficace d'au moins une huile siliconée phénylée de haute viscosité supérieure ou égale à 5.10⁻⁴ m²/s (500 cSt), d'au moins une huile hydrocarbonée non volatile ayant une masse moléculaire supérieure à 600 g/mol soluble ou dispersible dans ladite huile siliconée, l'huile hydrocarbonée non volatile étant choisie parmi les polyesters hydroxylés lesdits polyesters hydroxylés étant des polyesters de monoalcool gras et d'acide polycarboxylique hydroxylé.

Par "physiologiquement acceptable", on entend non toxique et susceptible d'être appliqué sur la peau (y compris l'intérieur des paupières), les lèvres ou les phanères d'êtres humains.

Par « au moins » un composé, on entend un ou plusieurs composés.

Par "huile", on entend tout milieu non aqueux liquide à température ambiante (25°C) et pression atmosphérique (760mm de Hg).

Par composé "non-volatil", on entend un composé susceptible de rester sur la peau ou les lèvres pendant plusieurs heures. Un composé non-volatil a en particulier une pression de vapeur, à température ambiante et pression atmosphérique, non nulle, inférieure à 0,02 mm de Hg (2,66 Pa).

Par composé "volatil", on entend un composé susceptible de s'évaporer de la peau ou des lèvres, en moins d'une heure. Un composé volatil est notamment choisi parmi les composés ayant une pression de vapeur, à température ambiante et pression atmosphérique allant de 0,02 mm à 300mm de Hg (2,66 Pa à 40 000 Pa) et mieux allant de 0,1 à 90 mm de Hg (13 Pa à 12 000 Pa).

Par huile siliconée phénylée "de haute viscosité", on entend une huile ayant une viscosité au moins égale à 5.10⁻⁴ m²/s(500 cSt) à 25°C mesurée selon la norme ASTM D-445. De préférence, l'huile siliconée n'est pas une gomme de silicone. Avantageusement, l'huile siliconée phénylée de haute viscosité présente une viscosité à 25 °C allant par exemple de 5.10⁻⁴ à 1.10⁻² m²/s (500 à 10 000 cSt), de préférence de 6.10⁻⁴ à 5.10⁻³ m²/s(600 à 5000 cSt), et mieux de 6.10⁻⁴ à 3.10⁻³ m²/s (600 à 3000 cSt)

De façon avantageuse, la composition selon l'invention comprend en outre une huile siliconée phénylée de basse viscosité qui présente une viscosité inférieure à 5.10⁻⁴ m²/s (500 cSt) à 25°C mesurée selon la norme ASTM D-445. De préférence, l'huile siliconée phénylée de basse viscosité présente une viscosité à 25°C allant par exemple de 5.10⁻⁶ à 499.10⁻⁶ m²/s (5 à 499 cSt), de préférence de 5.10⁻⁶ à 0.3⁻⁴m²/s de (5 à 300 cSt), et mieux de 5.10⁻⁶ à 1.10⁻⁴ (5 à 100 cSt).

L'huile siliconée phénylée de haute viscosité et l'huile siliconée phénylée de basse viscosité (si présente) peu(ven)t être par exemple une phényl trimethicone, une phényl dimethicone, une phényl triméthylsiloxy diphénylsiloxane, une diphényl diméthicone, une diphényl méthyldiphényl trisiloxane, ou un mélange de différentes huiles siliconées phénylées, et en particulier peu(ven)t répondre à la formule (A) suivante : 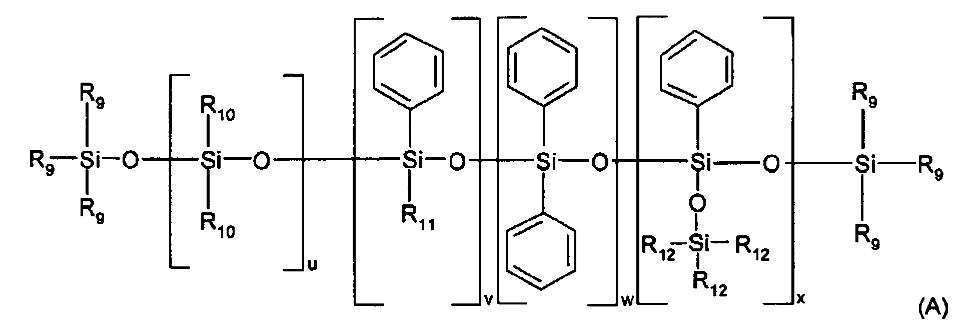 dans laquelle :
- R₉ et R₁₂ sont chacun indépendamment un radical alkyle en C₁-C₃₀, un radical aryle ou un radical aralkyle,
- R₁₀ et R₁₁ sont chacun indépendamment un radical alkyle en C₁-C₃₀ ou un radical aralkyle,
- u, v, w et x sont chacun indépendamment des nombres entiers allant de 0 à 900, sous réserve que la somme v+w+x soit différente de 0 et que la somme u+v+w+x va de 1 à 900 ; en particulier, u+v+w+x va de 1 à 800.
De préférence, v est égal à 0.

De préférence, l'huile siliconée phénylée de basse viscosité satisfait à la formule (A) avec la somme u+v+w+x allant de 1 à 150, et mieux de 1 à 100, voire de 1 à 50 et l'huile siliconée phénylée de haute viscosité satisfait à la formule (A) avec la somme u+v+w+x allant de 151 à 900, mieux de 160 à 800, voire de 160 à 500.

Parmi les huiles siliconées phénylées de haute viscosité utilisables dans l'invention, on peut citer les huiles 15 M 30 de PCR (5.10⁴ m²/s - 500 cSt) ou la Belsil PDM 1000 (1.10⁻³ m²/s- 1000 cSt) de Wacker.
Les valeurs entre parenthèses représentent les viscosités à 25°C.

Parmi les huiles siliconées phénylées de haute viscosité utilisables dans l'invention, on peut citer les huiles DC556 (2,25.10⁻⁵ m²/s -22,5 cSt) SF558 (1.10⁻⁵ à 2.10⁻⁵ m²/s- 10-20 cSt) de Dow Coming, l'huile Abil AV8853 (4.10-6 à 6.10-6 m²/s - 4-6 cSt) de Goldschmidt, l'huile Silbione 70 633 V 30 (2,8.10⁻⁵ m²/s - 28 cSt) de Rhône Poulenc, les huiles 15 M 40 (5.10⁻⁵ à 1.10⁻⁴ m²/s- 50 à 100 cSt), 15 M 50 (2 à 2,5.10⁻⁵- 20 à 25 cSt) de PCR, les huiles SF 1550 (215.10⁻⁶ m²/s- 25 cSt), PK 20 (2.10⁻⁵ m²/s - 20 cSt) de Bayer, les huiles Belsil PDM 200 (2.10⁻⁴ m²/s - 200 cSt) et Belsil PDM 20 (2.10⁻⁵ m²/s - 20 cSt) de Wacker, les huiles KF 53 (1,75.10⁻⁴ m²/s - 175 cSt), KF 54 (4.10⁻⁴m²/s - 400 cSt) et KF 56 (1.4.10⁻⁵ m²/s -14 cSt) de Shin-Etsu.

L'huile siliconée phénylée de haute viscosité peut représenter de 5 à 99% du poids total de la composition, de préférence de 7,5 à 80%, mieux de 10 à 60% et encore mieux de 20 à 50%.

L'huile siliconée phénylée de basse viscosité (si présente) peut représenter de 5 à 99% du poids total de la composition, de préférence de 7,5 à 80%, mieux de 10 à 60% et encore mieux de 10 à 40%.

Le rapport en poids entre l'huile siliconée phénylée de basse viscosité et l'huile siliconée phénylée de haute viscosité peut aller par exemple de 1/10 à 10/1, de préférence de 2/10 à 10/2, mieux de 3/10 à 10/5. De façon préférentielle, ce rapport en poids est égal à 1/3.

La composition selon l'invention comprend également au moins une huile hydrocarbonée non volatile ayant une masse moléculaire supérieure à 500 g/mol, de préférence supérieure à 600 g/mol, et mieux, supérieure à 650 g/mol, mais ne dépassant pas 15000 g/mol. L'indice de réfraction est de préférence supérieur à 1,440 à 20°C (l'indice de réfraction étant mesuré au réfractomètre), avantageusement supérieur à 1,450, et mieux, supérieur à 1,460.

Par composé "hydrocarboné", on entend un composé comportant principalement des atomes de carbone et d'hydrogène et éventuellement une ou plusieurs fonctions choisies parmi les fonctions hydroxyle, ester, éther, carboxylique. Ces composés sont en particulier exempts de groupements -Si-O-.

Cette huile hydrocarbonée non volatile peut être choisie parmi :
- les polymères lipophiles tels que :
   - les polybutylènes tels que l'INDOPOL H-100 (de masse molaire ou MM=965 g/mol), l'INDOPOL H-300 (MM=1340 g/mol), l'INDOPOL H-1500 (MM=2160g/mol) commercialisés ou fabriqués par la société AMOCO,
- les polyisobutylènes hydrogénés tels que le PANALANE H-300 E commercialisé ou fabriqué par la société AMOCO (M =1340 g/mol, indice de réfraction de 1,498), le VISEAL 20000 commercialisé ou fabriqué par la société SYNTEAL (MM=6000 g/mol), le

De préférence, l'huile hydrocarbonée non volatile est choisie parmi les esters d'acides gras linéaires ayant un nombre total de carbone allant de 30 à 70, les esters hydroxylés, les esters aromatiques, les esters d'alcool gras ou d'acides gras ramifiés en C24-C28 et leurs mélanges.

L'huile hydrocarbonée non volatile est de préférence choisie parmi les esters d'acides gras linéaires, les polyesters d'alcools gras et de polyacides éventuellement hydroxylés, et leurs mélanges.

De préférence encore, l'huile hydrocarbonée non volatile est choisie parmi les polyesters hydroxylés, notamment les polyesters de monoalcools gras et d'acide polycarboxylique hydroxylé. Le monoalcool gras comprend de préférence de 16 à 22 atomes de carbone et l'acide polycarboxylique est de préférence un acide dicarboxylique.

L'huile hydrocarbonée non volatile peut représenter de 5 à 99 %, de préférence de 10 à 60 % et mieux de 15 à 50 % du poids total de la composition.

La composition selon l'invention contient au moins un agent rhéologique structurant de son milieu physiologiquement acceptable.

Cet agent rhéologique est capable d'épaissir et/ou gélifier la composition. Il peut être présent en une quantité efficace pour augmenter la viscosité de la composition, notamment jusqu'à l'obtention d'un gel solide, à savoir un produit ne s'écoulant pas sous son propre poids. Il est ainsi possible d'obtenir un stick.
Cet agent rhéologique est avantageusement choisi parmi les cires, les composés gras pâteux à température ambiante (25°C), les gélifiants lipophiles et leurs mélanges.

L'agent rhéologique peut représenter de 0,1 à 65%, de préférence de 1 à 50%, mieux de 3 à 40% et encore mieux de 5 à 30% du poids total de la composition.

Par "cire" au sens de la présente invention, on entend un composé gras lipophile, solide à température ambiante (25°C), à changement d'état solide/liquide réversible, ayant une température de fusion supérieure à 30°C, et mieux supérieure à 45°C, pouvant aller jusqu'à 150° C, une dureté supérieure à 0,5 MPa à température ambiante, et présentant à l'état solide une organisation cristalline anisotrope. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Comme cires utilisable dans l'invention, on peut citer celles généralement utilisées dans le domaine cosmétique : elles sont notamment d'origine naturelle comme la cire d'abeilles, la cire de Carnauba, de Candelilla, d'Ouricoury, du Japon, de fibres de liège ou de canne à sucre, de riz, de Montan, la paraffine, les cires de lignite ou microcristallines, la cérésine ou l'ozokérite, les huiles hydrogénées comme l'huile de jojoba ou l'huile de ricin hydrogénée ; les cires synthétiques comme les cires de polyéthylène issues de la polymérisation ou copolymérisation de l'éthylène et les cires de Fischer-Tropsch ou encore des esters d'acides gras comme l'octacosanyl stéarate, les glycérides concrets à 30°C et mieux à 45°C, les cires de silicones comme les alkyl- ou alkoxydiméthicones ayant une chaîne alkyle ou alcoxy de 10 à 45 atomes de carbone, les esters de poly(di)méthylsiloxane solide à 30°C dont la chaîne ester comporte au moins 10 atomes de carbone ou encore le tétrastéarate de di-(triméthylol-1,1,1 propane) fabriqué ou commercialisé par la société Hétérène sous la dénomination HEST 2T- 4S et leurs mélanges.

De préférence, l'agent rhéologique comprend un mélange de cire microcristalline et de cire siliconée, telle que la cire alkyldimethicone ayant une chaîne alkyle en C30-C45.

La ou les cires peuvent être présentes en une quantité allant de 0,1 à 50% en poids par rapport au poids total de la composition, mieux de 1 à 30% et mieux de 3 à 25%.

L'agent rhéologique peut aussi être un composé gras pâteux à température ambiante (25°C). Par "composé gras pâteux", on entend un corps gras ayant une dureté, mesurée à température ambiante, allant de 0,001 à 0,5 MPa, de préférence allant de 0,005 à 0,4 MPa. Un pâteux a en outre un point de fusion compris entre 20 et 60°C, de préférence allant de 25 à 45°C.
Un composé pâteux est un produit visqueux comprenant une fraction solide et une fraction liquide.

De préférence, ces corps gras sont des composés hydrocarbonés, éventuellement de type polymérique ; ils peuvent également être choisis parmi les composés siliconés et/ou fluorés ; ils peuvent aussi se présenter sous forme d'un mélange de composés hydrocarbonés et/ou siliconés et/ou fluorés. Dans le cas d'un mélange de différents corps gras pâteux, on utilise de préférence les composés pâteux hydrocarbonés en proportion majoritaire.

Parmi les composés pâteux susceptibles d'être utilisés dans la composition selon l'invention, on peut citer les lanolines et les dérivés de lanoline comme les lanolines acétylées, le lanolate de magnésium ou les lanolines oxypropylènées et leurs mélanges. On peut également utiliser des esters d'acides gras ou d'alcools gras, notamment ceux ayant de 20 à 65 atomes de carbone (point de fusion de l'ordre de 20 à 35°C) comme le citrate de tri-isostéaryle, le propionate d'arachidyle, le polylaurate de vinyle ; les esters du cholestérol ; les triglycérides d'origine végétale tels que les huiles végétales hydrogénées, les polyesters visqueux comme l'acide poly(12-hydroxystéarique) et leurs mélanges. Comme triglycérides d'origine végétale, on peut utiliser les dérivés d'huile de ricin hydrogénée, tels que le « THIXINR » de Rheox ou encore le mélange de triglycérides d'acides laurique, myristique, palmitique et stéarique fabriqué ou commercialisé sous la référence Softisan 100 par la société Sasol.

On peut aussi citer les corps gras pâteux siliconés tels que les polydiméthylsiloxanes (PDMS) ayant des chaînes pendantes du type alkyle ou alcoxy ayant de 8 à 24 atomes de carbone, et un point de fusion de 20-55°C, comme les stearyl dimethicones notamment ceux vendus par la société Dow Corning sous les noms commerciaux de DC2503 et DC25514, et leurs mélanges.

Le ou les corps gras pâteux peuvent être présents à raison de 0,1 à 60% en poids, par rapport au poids total de la composition, de préférence à raison de 1-45% en poids et encore plus préférentiellement à raison de 2-30% en poids, dans la composition, s'ils sont présents.

Le gélifiant lipophile peut être organique ou minéral, polymérique ou moléculaire. Comme gélifiant lipophile minéral, on peut citer les argiles éventuellement modifiées comme les hectorites modifiées par un chlorure d'ammonium d'acide gras en C₁₀ à C₂₂, comme l'hectorite modifiée par du chlorure de di-stéaryl di-méthyl ammonium ; la silice pyrogénée éventuellement traitée hydrophobe en surface dont la taille des particules est inférieure à 1µm. Les gélifiants lipophiles organiques polymériques sont par exemple les organopolysiloxanes élastomériques partiellement ou totalement réticulés, de structure tridimensionnelle, comme ceux commercialisés sous les noms KSG6, KSG16, KSG18 de Shin-Etsu, Trefil E-505C ou Trefil E-506C de Dow-Corning, Gransil SR-CYC, SR DMF10, SR-DC556, SR 5CYC gel, SR DMF 10 gel, SR DC 556 gel de Grant Industries, SF 1204 et JK 113 de General Electric ; l'éthylcellulose comme celles vendues sous le nom d'Ethocel par Dow Chemical ; les copolymères d'un diacide en C₃₆ condensé sur l'éthylène diamine de masse moléculaire moyenne en poids d'environ 6000 tels que les composés commercialisés par la société Arizona Chemical sous les noms Uniclear 80 et Uniclear 100, les gommes notamment siliconées comme les PDMS ayant une viscosité > 10.000 cm².s-1 (100 000 centistokes) les galactommananes comportant de un à six et mieux de deux à quatre groupes hydroxyle par ose, substitués par une chaîne alkyle saturée ou non, comme la gomme de guar alkylée par des chaînes alkyle en C₁ à C₆ et mieux en C₁ à C₃ et leurs mélanges.

Comme gélifiants lipophiles préférés, on utilise des gélifiants organiques moléculaires non polymériques, également appelés organogélateurs, qui sont des composés dont les molécules sont capables d'établir entre elles des interactions physiques conduisant à une auto-agrégation des molécules avec formation d'un réseau supra-moléculaire 3D qui est responsable de la gélification de la phase grasse liquide.
Par "phase grasse liquide", on entend, au sens de l'invention, une phase grasse liquide à température ambiante (25° C) et pression atmosphérique (760 mm de Hg, soit 105 Pa), composée d'un ou plusieurs corps gras liquides température ambiante, appelés aussi huiles, généralement compatibles entre eux. En particulier, cette phase grasse liquide est constituée par les huiles hydrocarbonées non volatiles décrites précédemment.

Le réseau supra-moléculaire peut résulter de la formation d'un réseau de fibrilles (dues aux empilements ou agrégations de molécules d'organogélateur), immobilisant les molécules de la phase grasse liquide.
L'aptitude à former ce réseau de fibrilles, et donc à gélifier, dépend de la nature (ou classe chimique) de l'organogélateur, de la nature des substituants portés par ses molécules pour une classe chimique donnée et de la nature de la phase grasse liquide.
Les interactions physiques sont diverses mais excluent la co-cristallisation. Ces interactions physiques sont en particulier des interactions du type interactions hydrogènes auto-complémentaires, interactions π entre cycles insaturés, interactions dipolaires, liaisons de coordination avec des dérivés organométalliques et leurs associations. En général, chaque molécule d'un organogélateur peut établir plusieurs types d'interactions physiques avec une molécule voisine. Aussi, avantageusement, les molécules des organogélateurs selon l'invention comportent au moins un groupement capable d'établir des liaisons hydrogène et mieux au moins deux groupements capables d'établir des liaisons hydrogène, au moins un cycle aromatique et mieux aux moins deux cycles aromatiques, au moins une ou plusieurs liaisons à insaturation éthylénique et/ou au moins un ou plusieurs carbones asymétriques. De préférence, les groupements capables de faire des liaisons hydrogènes sont choisis parmi les groupements hydroxyle, carbonyle, amine, acide carboxylique, amide, urée, benzyle et leurs associations.

Le ou les organogélateurs selon l'invention sont solubles dans la phase grasse liquide après chauffage jusqu'à obtention d'une phase liquide homogène transparente. Ils peuvent être solides ou liquides à température ambiante et pression atmosphérique.

Le ou les organogélateurs moléculaires utilisables dans la composition selon l'invention sont notamment ceux décrits dans le document "Specialist Surfactants", édité par D. Robb de 1997, p.209-263, chapitre 8 de P. Terech, les demandes européennes EP-A-1068854 et EP-A-1086945 ou encore dans la demande WO-A-02/47031.

On peut notamment citer parmi ces organogélateurs, les amides d'acides carboxyliques en particulier les acides tri-carboxyliques comme les cyclohexanetricarboxamides (voir la demande de brevet européen EP-A-1068854), les diamides ayant des chaînes hydrocarbonées contenant chacune de 1 à 22 atomes de carbone, par exemple de 6 à 18 atomes de carbone, lesdites chaînes étant non substituées ou substituées avec au moins un substituant choisi parmi les groupes ester, urée et fluoro (voir la demande EP-A-1086945) et notamment les diamides résultant de la réaction du diaminocyclohexane, en particulier du diaminocyclohexane sous forme trans, et d'un chlorure d'acide comme par exemple le N,N'-bis (dodécanoyl)-1,2-diaminocyclohexane, les amides de N-acylamino acides comme les diamides résultant de l'action d'un N-acylamino acide avec des amines comportant de 1 à 22 atomes de carbone, comme par exemple ceux décrits dans le document WO-93/23008 et notamment les amides de l'acide N-acylglutamique où le groupe acyle représente une chaîne alkyle en C₈ à C₂₂ tels que le dibutylamide de l'acide N-Lauroyl-L-glutamique, fabriqué ou commercialisé par la société Ajinomoto sous la dénomination GP-1 et leurs mélanges.

Le gélifiant lipophile peut représenter de 0,1 à 50 % en poids, de préférence de 1 à 30 % en poids, et mieux de 2 à 20 % en poids par rapport au poids total de la composition.

La phase particulaire présente dans la composition selon l'invention comprend des pigments et/ou des nacres et/ou des charges habituellement utilisés dans les compositions cosmétiques.
La phase particulaire est généralement présente à raison de 0,01 à 60%, de préférence 10 à 5 à 25% en poids par rapport au poids total de la composition.

Selon l'invention, l'agent rhéologique est distinct de la phase particulaire et en particulier de la ou des charges éventuellement présents dans la phase particulaire.

Par pigments, il faut comprendre des particules blanches ou colorées, minérales ou organiques, insolubles dans les corps gras tels que les huiles, destinées à colorer et/ou opacifier la composition. Par charges, il faut comprendre des particules incolores ou blanches, minérales ou de synthèse, lamellaires ou non lamellaires. Par nacres ou pigments nacrés, il faut comprendre des particules irisées, notamment produites par certains mollusques dans leur coquille ou bien synthétisées.

Les pigments peuvent être blancs ou colorés, minéraux et/ou organiques, enrobés ou non, sphériques ou oblongues. On peut citer, parmi les pigments minéraux, le dioxyde de titane, éventuellement traité en surface, les oxydes de zirconium ou de cérium, ainsi que les oxydes de zinc, de fer (noir, jaune, brun ou rouge) ou de chrome, le violet de manganèse, le bleu outremer, l'hydrate de chrome et le bleu ferrique. Parmi les pigments organiques, on peut citer le noir de carbone, les pigments de type laques organiques de baryum, strontium, calcium ou aluminium ou encore les laques à base de carmin de cochenille. Les pigments peuvent être présents dans la composition à raison de 0,05 à 40% du poids de la composition finale, et de préférence à raison de 2 à 20 % pour une composition non pulvérulente.

Les nacres ou pigments nacrés peuvent être choisis parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth. On peut aussi utiliser des pigments à propriétés goniochromatiques, notamment à cristaux liquides ou multicouches. Les nacres peuvent être présentes dans la composition à raison de 0,01 à 20 % du poids total de la composition, de préférence à un taux de l'ordre de 1 à 15 %.

Les charges peuvent être présentes à raison de 0,01 à 35 % (si présentes) du poids total de la composition, de préférence 0,5 à 15 %. On peut notamment citer le talc, le mica, le kaolin, la lauroyl lysine, les poudres de polyamide telles que le Nylon® (Orgasol notamment) et de polyéthylène, les poudres de polytétrafluoroéthylène (Téflon®), l'amidon, le nitrure de bore, des microsphères de copolymères telles que l'Expancel® (Nobel Industrie), le Polytrap® (Dow Corning), le Polypore® L 200 (Chemdal Corporation) et les microbilles de résine de silicone (Tospearl ® de Toshiba, par exemple).

De préférence, la composition de l'invention contient peu ou pas d'huiles volatiles et notamment moins de 10 % par rapport au poids total de la composition, de préférence moins de 5 % et mieux moins de 2 % et avantageusement est exempte d'huile volatile.

La composition selon l'invention peut comprendre en outre au moins un composé non aqueux additionnel différent de l'huile siliconée phénylée et de l'huile hydrocarbonée non volatile de masse moléculaire supérieur à 500 g/mol choisi parmi les huiles, des gommes, des résines, des polymères lipophiles et leurs mélanges.

Les gommes utilisables dans l'invention se présentent généralement sous forme solubilisée dans une huile et les résines peuvent être liquides ou solides à température ambiante.

La nature et la quantité des gommes et des résines sont fonction des propriétés mécaniques et des textures recherchées.

Les huiles additionnelles peuvent être des huiles hydrocarbonées et/ou siliconées et/ou fluorées. Ces huiles peuvent être d'origine animale, végétale, minérale ou synthétique. A titre d'exemple d'huile additionnelle utilisable dans l'invention, on peut citer :
- les huiles hydrocarbonées d'origine animale telles que le perhydrosqualène ;
- les huiles hydrocarbonées végétales telles que les triglycérides liquides d'acides gras ayant de 4 à 10 atomes de carbone comme les triglycérides des acides heptanoïque ou octanoïque ou l'huile de jojoba ;
- les hydrocarbures linéaires ou ramifiés, d'origine minérale ou synthétique tels que les huiles de paraffine et leurs dérivés, la vaseline ;
- les esters et les éthers de synthèse notamment d'acides gras comme les huiles de formule R₁COOR₂ dans laquelle R₁ représente le reste d'un acide gras supérieur comportant de 1 à 40 atomes de carbone et R₂ représente une chaîne hydrocarbonée contenant de 1 à 40 atomes de carbone avec 10 = R₁ + R₂ = 41 comme par exemple, , le myristate d'isopropyle, le palmitate d'éthyl 2-hexyle, le stéarate d'octyl 2-dodécyle, l'érucate d'octyl 2-dodécyle ;
- les alcools gras ayant de 12 à 26 atomes de carbone comme l'octyl dodécanol, le 2-butyloctanol, le 2-hexyl décanol, le 2-undécyl pentadécanol, l'alcool oléique ;
- les acides gras supérieurs en C₈-C₂₆ tels que l'acide oléique, l'acide linoléique, l'acide linolénique ou l'acide isostéarique ;
- les huiles fluorées éventuellement partiellement hydrocarbonées et/ou siliconées ;
- les huiles siliconées comme les polydiméthylsiloxanes (PDMS) volatiles ou non, linéaires ou cycliques ; les polydiméthylsiloxanes comportant des groupements alkyle ou alcoxy, pendant ou en bout de chaîne siliconée, groupements ayant de 2 à 24 atomes de carbone ;
- et leurs mélanges.

La composition de l'invention peut comprendre, en outre, au moins un additif usuellement utilisé dans le domaine concerné, tel que de l'eau, des colorants, des arômes, des parfums, des antioxydants, des conservateurs, des neutralisants, des gélifiants de phase aqueuse, des dispersants, des actifs cosmétiques, et leurs mélanges. Cet additif, à l'exception de l'eau qui peut représenter de 0,01 à 80 % et par exemple de 1 à 70 et mieux de 1 à 60 % du poids total de la composition, peut être présent dans la composition à raison de 0,0005 à 20% du poids total de la composition et mieux de 0,001 à 10%.

Par « actif cosmétique », on entend un composé lipophile ou hydrophile apportant un bénéfice aux matières kératiniques et plus spécialement à la peau et aux lèvres.

Comme actif cosmétique utilisable dans l'invention, on peut citer les vitamines A, E, C, B₃, F, les provitamines comme le D-panthénol, les actifs apaisants comme l'α-bisabolol, l'aloe vera, l'allantoïne, les extraits de plantes ou les huiles essentielles, les agents protecteurs ou restructurants comme les céramides, les actifs fraîcheur comme le menthol et ses dérivés, les émollients (beurre de cacao, diméthicone), les hydratants (arginine PCA), les actifs antirides, les acides gras essentiels, les filtres solaires, et leurs mélanges.

Bien entendu l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée.

Les applications de la composition selon l'invention sont multiples et concernent l'ensemble des produits cosmétiques colorés ou non et plus particulièrement les produits de maquillage des lèvres tels que les rouges à lèvres ou les brillants à lèvres ou encore les crayons à lèvres.

Dans un mode de réalisation particulier de l'invention, la composition selon l'invention peut se présenter sous forme d'un produit coulé, et par exemple sous la forme d'un stick ou bâton, ou sous la forme de coupelle utilisable par contact direct ou à l'éponge ou encore dans une bouillotte. En particulier, elle se présente sous forme solide et trouvent alors une application en tant que fond de teint coulé, fard à joues ou à paupières coulé, rouge à lèvres, base ou baume de soin pour les lèvres, produit anti-cernes. Elle peut aussi se présenter sous forme un fond de teint ou un rouge à lèvres fluides, un brillant à lèvres (gloss en terminologie anglo-saxonne), un produit solaire ou de coloration de la peau.

La composition de l'invention peut être anhydre et contenir moins de 5 % d'eau ajoutée par rapport au poids total de la composition. Elle peut alors se présenter notamment sous forme de gel huileux, de liquide huileux, de pâte ou de stick anhydre contenant notamment une dispersion vésiculaire de lipides ioniques et/ou non ioniques.
Elle peut aussi se présenter sous forme d'une émulsion simple ou multiple à phase continue huileuse ou aqueuse, de dispersion huileuse dans une phase aqueuse grâce à des vésicules contenant des lipides ioniques et/ou non ioniques. Ces formes galéniques sont préparées selon les méthodes usuelles des domaines considérés.
La composition selon l'invention peut se présenter sous la forme d'une composition, colorée ou non, de soin de la peau, sous forme d'une composition de protection solaire ou de démaquillage ou encore sous forme d'une composition hygiénique. Si elle contient des actifs cosmétiques, elle peut alors être utilisée comme base de soin ou de traitement non thérapeutique pour la peau comme les mains ou le visage ou pour les lèvres (baumes à lèvres, protégeant les lèvres du froid et/ou du soleil et/ou du vent), produit de bronzage artificiel de la peau.

La composition de l'invention peut également se présenter sous la forme d'un produit de maquillage coloré de la peau, en particulier du visage comme un blush, un fard à joues ou à paupières, de maquillage du corps comme un produit de tatouage semi-permanent ou de maquillage des lèvres comme un rouge ou un brillant à lèvres, présentant éventuellement des propriétés de soin ou de traitement non thérapeutique, un produit de maquillage des phanères comme par exemple un vernis à ongles, un mascara, un eyeliner, un produit de coloration ou de soin des cheveux.

De préférence, la composition selon l'invention se présente sous la forme d'un produit de maquillage des lèvres tel qu'un rouge à lèvres ou un brillant à lèvres.

Un produit de maquillage des lèvres se présente avantageusement sous forme anhydre.

Bien entendu la composition de l'invention doit être physiologiquement acceptable, à savoir non toxique et susceptible d'être appliquée sur la peau (y compris l'intérieur des paupières), les lèvres ou les phanères d'êtres humains. Elle est en particulier cosmétiquement acceptable, c'est à dire agréable de goût, de toucher, d'aspect et/ou d'odeur, applicable plusieurs fois par jour pendant plusieurs mois.

La composition selon l'invention peut être fabriquée par les procédés connus, généralement utilisés dans le domaine cosmétique.

Les exemples qui suivent ont pour but d'illustrer l'objet de la présente invention. Les quantités sont données en pourcentage massique.

### Exemples 1 à 4 : Essais comparatifs - Sticks de Rouges à Lèvres

Les compositions figurant dans le tableau (1 ) ci-après ont été réalisées :
- la composition de l'exemple 1 comprend une huile siliconée, le cyclopentasiloxane,
- la composition de l'exemple 2 comprend une huile hydrocarbonée volatile, l'isododécane de masse moléculaire 170 g/mol,
- la composition de l'exemple 3 comprend une huile hydrocarbonée non volatile, l'isononanoate d'isononyle ayant une masse moléculaire égale à 284 g/mol et un indice de réfraction de 1,436,
- la composition de l'exemple 4 selon l'invention comprend du diisostéaryl malate ayant une masse moléculaire égale à 639 g/mol et un indice de réfraction de 1,462.

**Tableau (1)**

| **Phase** | | **Exemple 1 (comparatif)** | **Exemple 2 (comparatif)** | **Exemple 3 (comparatif)** | **Exemple 4 (invention)** |
|---|---|---|---|---|---|
| **A** | Cyclopentasiloxane | 30 | | | |
| | Isododécane | | 30 | | |
| | Isononanoate d'isononyle | | | 30 | |
| | Malate de di-isostéaryle | | | | 30 |
| | Phényltrimethyltrisiloxane (2.10⁻⁵ m²/s - 20 cSt) fabriquée ou commercialisée par la société Dow Corning sous la référence DC 556 | 18 | 18 | 18 | 18 |
| | Phényltrimethyltrisiloxane (1.10⁻³ m²/s - 1000 cSt) fabriquée ou commercialisée par la société Wacker sous la référence Belsil PDM 1000 | Qsp 100 | Qsp 100 | Qsp 100 | Qsp 100 |
| **B** | Cire microcristalline | 10 | 10 | 10 | 10 |
| | Alkyl diméthicone en C₃₀-C₄₅ | 2,5 | 2,5 | 2,5 | 2,5 |
| | Mélange de triglycérides d'acides laurique, myristique, palmitique et stéarique (50/20/10/10) fabriqué ou commercialisé sous la référence Softisan 100 par la société Sasol. | 10 | 10 | 10 | 10 |
| **C** | Red 7 | 0,26 | 0,26 | 0,26 | 0,26 |
| | Red 21 | 0,06 | 0,06 | 0,06 | 0,06 |
| | Oxyde de fer noir | 0,09 | 0,09 | 0,09 | 0,09 |
| | Oxyde de fer brun | 2,1 | 2,1 | 2,1 | 2,1 |
| | Mica oxyde de titane | 1,8 | 1,8 | 1,8 | 1,8 |

### Mode opératoire

Les pigments (phase C) sont broyés dans le malate de diisostéaryle de la phase A, puis le broyat est mélangé à la phase (B) (cires et pâteux) et au restant de la phase A. Le mélange est chauffé dans un poêlon double enveloppe pendant au moins 30 minutes après fonte totale des cires.

La pâte obtenue est coulée dans un moule approprié pour stick qui est porté à 40-42 °C et qui est ensuite placé à -18°C pendant une demi-heure. Puis les sticks sont démoulés.

### Evaluation cosmétique

Les 4 sticks de rouge à lèvres ont été évalués par 5 personnes qualifiées selon différents critères.

Les sticks des exemples 2 et 3 ont été jugés comme ayant de mauvaises propriétés de dépôt car de consistance trop molle ; le stick de l'exemple 1 a été jugé comme déposant bien sur les lèvres mais présentant une perte de brillance dans le temps.
Le stick de l'exemple 4 selon l'invention a été jugé comme déposant bien, le film de composition a été jugé homogène et brillant.

### Exemple 5 : Stick de Rouge à lèvres

| Phase | | |
|---|---|---|
| A | Malate de di-isostéaryle | qsp 100 |
| | Phényltrimethyltrisiloxane (2.10⁻⁵ m²/s - 20 cSt) fabriquée ou commercialisée par la société Dow Corning sous la référence DC 556 | 18 |
| | Phényltrimethyltrisiloxane (1.10⁻³ m²/s -1000 cSt) fabriquée ou commercialisée par la société Wacker sous la référence Belsil PDM 1000 | 27 |
| B | Cire microcristalline | 10 |
| | Alkyl diméthicone en C30-C45 | 2,5 |
| | Mélange de triglycérides d'acides laurique, myristique, palmitique et stéarique (50/20/10/10) fabriqué ou commercialisé sous la référence Softisan 100 par la société Sasol. | 10 |
| C | Red 7 | 0,26 |
| | Red 21 | 0,06 |
| | Oxyde de fer noir | 0,09 |
| | Oxyde de fer brun | 2,1 |
| | Mica oxyde de titane | 1,8 |

Le mode opératoire est le même que celui des exemples 1 à 4.

### Evaluation cosmétique :

La tenue de cette composition a été évaluée à l'aide de méthodes instrumentales et sensorielles sur un panel de 12 personnes qualifiées qui ont appliqué le stick de rouge à lèvres.

L'évaluation de la tenue se déroule de la façon suivante :
- dans un premier temps, une évaluation de la tenue "sensorielle" globale est réalisée une heure après l'application de la formule sur les lèvres.
- dans un second temps, une tenue "instrumentale" est évaluée après une série d'épreuves consistant à faire deux « bises » sur un mouchoir en papier, boire une boisson chaude puis une boisson froide et manger 4 bouchées d'un sandwich et d'une pomme.

La tenue sensorielle est évaluée sur une échelle allant de 1 à 10 : 1 correspond à une formule qui ne tient pas du tout et 10 à une formule qui tient très bien.
La tenue instrumentale est évaluée sur une échelle allant de 1 à 100 : 1 correspond à une formule qui ne tient pas du tout et 100 à une formule qui tient très bien.

La brillance et le confort ont aussi été évalués par ces 12 personnes :
- la brillance a été évaluée juste après l'application de la formule puis au bout d'une heure (la brillance instrumentale est évaluée sur une échelle allant de 1 à 200. 1 correspond à une formule qui n'est pas du tout brillante et 200 à une formule qui est très brillante.
   La brillance instrumentale est mesurée à l'aide d'un brillancemètre, de manière conventionnelle, par la méthode suivante.
   Sur une carte de contraste de marque LENETA et de référence FORM 1A PENOPAC, on étale une couche de 50 µm d'épaisseur de la composition dont on cherche à évaluer la brillance moyenne, à l'aide d'un étaleur automatique. La couche recouvre au moins le fond blanc de la carte. On procède de suite à la mesure de la brillance à 20° sur le fond blanc à l'aide d'un brillancemètre de marque BYK GARDNER et de référence microTRl-GLOSS.
- le confort a été évalué au bout d'une heure.

### Résultats

| | | **Tenue** | **Brillance** | **Confort** |
|---|---|---|---|---|
| **Evaluation sensorielle** | A l'application | | 6,3 | |
| | A 1 heure | 6,4 | 4,8 | 7,4 |
| **Evaluation instrumentale** | A l'application | | 171 | |
| | A 1 heure | 82 | 138 | |
| | Après épreuves | 54 | | |

La composition présente de bonnes propriétés cométiques, notamment de brillance et de confort, et sa tenue dans le temps est très satisfaisante.
L'application (facilité d'application et glissant) du film de composition a également été jugée comme satisfaisante.

## Revendications

1. Composition cosmétique comprenant, dans un milieu physiologiquement acceptable, a) au moins une huile siliconée phénylée de haute viscosité présentant une viscosité à 25 °C allant de 5.10⁻⁴ à 1.10⁻² m²/s(500 à 10 000 cSt), b) au moins une huile hydrocarbonée non volatile ayant une masse moléculaire supérieure à 600 g/mol choisie parmi les polyesters hydroxylés, lesdits polyesters hydroxylés étant des polyesters de monoalcool gras et d'acide polycarboxylique hydroxylé, l'huile siliconée phénylée de haute viscosité étant choisie parmi les huiles de formule (A) suivante : 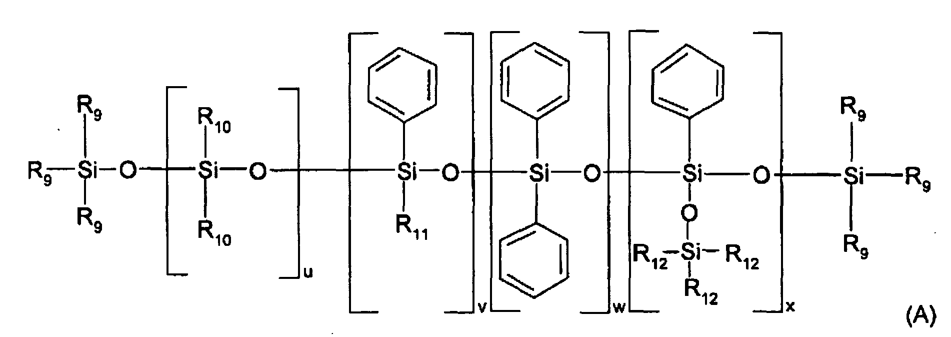 dans laquelle :
- R₉ et R₁₂ sont chacun indépendamment un radical alkyle en C₁-C₃₀, un radical aryle ou un radical aralkyle,
- R₁₀ et R₁₁ sont chacun indépendamment un radical alkyle en C₁-C₃₀ ou un radical aralkyle,
- u, v, w et x sont chacun indépendamment des nombres entiers allant de 0 à 900, sous réserve que la somme v+w+x soit différente de 0 et que la somme u+v+w+x va de 1 à 900, en particulier, u+v+w+x va de 1 à 800 et c) une phase particulaire, **caractérisée en ce qu'**elle comprend moins de 5% d'une huile volatile.

2. Composition selon l'une des revendications précédentes, **caractérisée en ce que** l'huile hydrocarbonée non volatile est le malate de diisostéaryle.

3. Composition selon l'une des revendications précédentes, **caractérisée en ce que** l'huile hydrocarbonée non volatile représente de 5 à 99 %, de préférence de 10 à 60 % et mieux de 15 à 50 % du poids total de la composition.

4. Composition selon l'une des revendications précédentes, **caractérisée en ce que** l'huile siliconée phénylée de haute viscosité présente une viscosité à 25°C allant de 6.10⁻⁴ à 5.10 m²/s(600 à 5000 cSt).

5. Composition selon l'une des revendications précédentes, **caractérisée en ce que** l'huile siliconée phénylée de haute viscosité présente une viscosité à 25°C allant de 6.10⁻⁴ à 3.10⁻³ m²/s (600 à 3000 cSt).

6. Composition selon l'une des revendications précédentes, **caractérisée en ce que** l'huile siliconée phénylée de haute viscosité représente de 5 à 99% en poids de la composition.

7. Composition selon l'une des revendications précédentes, **caractérisée en ce qu'**elle contient en outre une huile siliconée phénylée de basse viscosité à 25 °C inférieure à 5.10⁻⁴ (500 cSt).

8. Composition selon la revendication précédente, **caractérisée en ce que** l'huile siliconée phénylée de basse viscosité présente une viscosité à 25°C allant de 5.10⁻⁶ à 5.10⁻⁴m²/s (5 à 500 cSt).

9. Composition selon la revendication 7 ou 8, **caractérisée en ce que** l'huile siliconée phénylée de basse viscosité présente une viscosité à 25°C allant de 5.10⁻⁶ à 3.10⁻⁴ m²/s (5 à 300 cSt) à 25°C.

10. Composition selon l'une quelconque des revendications 7 à 9, **caractérisée en ce que** l'huile siliconée phénylée de basse viscosité présente une viscosité à 25°C allant de 5.10⁻⁶ à 1.10⁻⁴ m²/s (5 à 100 cSt) à 25°C.

11. Composition selon l'une des revendications 7 ou 8, **caractérisée en ce que** l'huile siliconée phénylée de basse viscosité est choisie parmi les huiles de formule (A) telle que définie en revendication 1.

12. Composition selon l'une des revendications 7 à 11, **caractérisée en ce que** l'huile siliconée phénylée de basse viscosité représente de 5 à 99% en poids de la composition.

13. Composition selon l'une des revendications 7 à 12, **caractérisée en ce que** le rapport en poids entre l'huile siliconée phénylée de basse viscosité et l'huile siliconée de haute viscosité va de 1/10 à 10/1.

14. Composition selon l'une des revendications7 à 13, **caractérisée en ce que** le rapport en poids entre l'huile siliconée phénylée de basse viscosité et l'huile siliconée de haute viscosité va de 2/10 à 10/2.

15. Composition selon l'une des revendications 7 à 14, **caractérisée en ce que** le rapport en poids entre l'huile siliconée phénylée de basse viscosité et l'huile siliconée de haute viscosité va de 3/10 à 10/5.

16. Composition selon l'une des revendications précédentes, **caractérisée en ce qu'**elle comprend en outre un agent rhéologique choisi parmi les cires, les composés gras pâteux à température ambiante de 25°C, les gélifiants lipophiles et leurs mélanges.

17. Composition selon la revendication précédente, **caractérisée en ce que** l'agent rhéologique représente de 0,1 à 65%, de préférence de 1 à 50%, mieux de 3 à 40% et encore mieux de 5 à 30% du poids total de la composition.

18. Composition selon la revendication 16ou 17, **caractérisée en ce que** l'agent rhéologique est une cire siliconée choisie parmi les alkyldiméthicones ou alkoxydiméthicones ayant une chaîne alkyle ou alcoxy de 10 à 45 atomes de carbone, les esters de poly(di)méthylsiloxane solide à 30°C dont la chaîne ester comporte au moins 10 atomes de carbone et leurs mélanges.

19. Composition selon l'une des revendications précédentes, **caractérisée en ce que** l'agent rhéologique est une cire hydrocarbonée apolaire choisie parmi la paraffine, les cires de lignite ou microcristallines, la cérésine ou l'ozokérite, les cires synthétiques comme les cires de polyéthylène issues de la polymérisation ou copolymérisation de l'éthylène, les cires de Fischer-Tropsch ou encore le tétrastéarate de di-(triméthylol-1,1,1 propane) et leurs mélanges.

20. Composition selon la revendication 16, **caractérisée en ce que** le gélifiant lipophile est un organogélateur.

21. Composition selon la revendication précédente, **caractérisée en ce que** l'organogélateur est choisi parmi les amides d'acides tri-carboxyliques, les diamides ayant des chaînes hydrocarbonées contenant chacune de 1 à 22 atomes de carbone, lesdites chaînes étant non substituées ou substituées avec au moins un substituant choisi parmi les groupes ester, urée et fluoro, les amides de N-acylamino acides et leurs mélanges.

22. Composition selon l'une des revendications 20 ou 21, **caractérisée en ce que** l'organogélateur est choisi parmi les cyclohexanetricarboxamides, les diamides résultant de la réaction du diaminocyclohexane et d'un chlorure d'acide, les diamides résultant de l'action d'un N-acylamino acide avec des amines comportant de 1 à 22 atomes de carbone et leurs mélanges.

23. Composition selon l'une des revendications 16 et 20 à 22, **caractérisée en ce que** le gélifiant lipophile représente de 0,1 à 50%, mieux 1 à 30 %, et mieux de 2 à 20 % en poids par rapport au poids total de la composition.

24. Composition selon l'une des revendications précédentes, **caractérisée en ce que** la phase particulaire comprend des pigments et/ou des nacres et/ou des charges.

25. Composition selon l'une des revendications précédentes, **caractérisée en ce que** la phase particulaire est présente à raison de 0,01 à 60%, de préférence 5 à 25% en poids par rapport au poids total de la composition.

26. Composition selon l'une des revendications précédentes, **caractérisée en ce qu'**elle se présente sous la forme d'un produit de maquillage et/ou de soin du visage ou du corps, des lèvres et/ou des phanères.

27. Composition selon l'une des revendications précédentes, **caractérisée en ce qu'**elle se présente sous la forme d'un produit de maquillage des lèvres.

28. Composition selon la revendication précédente, **caractérisée en ce qu'**elle se présente sous forme anhydre.

29. Utilisation de l'association d'au moins une huile siliconée phénylée de haute viscosité supérieure ou égale à 5.10⁻⁴ m²/s (500 cSt), d'au moins une huile hydrocarbonée non volatile, de masse moléculaire supérieure à 600 g/mol soluble ou dispersible dans ladite huile siliconée l'huile hydrocarbonée non volatile étant choisie parmi les polyesters hydroxylés, lesdits polyesters hydroxylés étant des polyesters de monoalcool gras et d'acide polycarboxylique hydroxylé, dans une composition comprenant un milieu physiologiquement acceptable et une phase particulaire et comprenant moins de 5 % d'une huile volatile, ladite composition présentant des propriétés de tenue et de brillance.

30. Procédé cosmétique pour conférer à un film de composition cosmétique comprenant une phase particulaire et moins de 5 % d'une huile volatile des propriétés de tenue et de brillance, consistant à introduire dans ladite composition une quantité efficace d'au moins une huile siliconée phénylée de haute viscosité supérieure ou égale à 5.10⁻⁴ m²/s (500 cSt), d'au moins une huile hydrocarbonée non volatile ayant une masse moléculaire supérieure à 600 g/mol soluble ou dispersible dans ladite huile siliconée l'huile hydrocarbonée non volatile choisie parmi les polyesters hydroxylés, lesdits polyesters hydroxylés étant des polyesters de monoalcool gras et d'acide polycarboxylique hydroxylé.

## Claims

1. Cosmetic composition comprising in a physiologically acceptable medium a) at least one high-viscosity phenylsilicone oil having a viscosity at 25°C of from 5 x 10⁻⁴ to 1 x 10⁻² m²/s (500 to 10 000 cSt), b) at least one non-volatile hydrocarbon oil having a molecular mass of more than 600 g/mol selected from hydroxyl-containing polyesters, the said hydroxyl-containing polyesters being polyesters of fatty monoalcohol and hydroxyl-containing polycarboxylic acid, the high-viscosity phenylsilicone oil being selected from the oils of following formula (A): 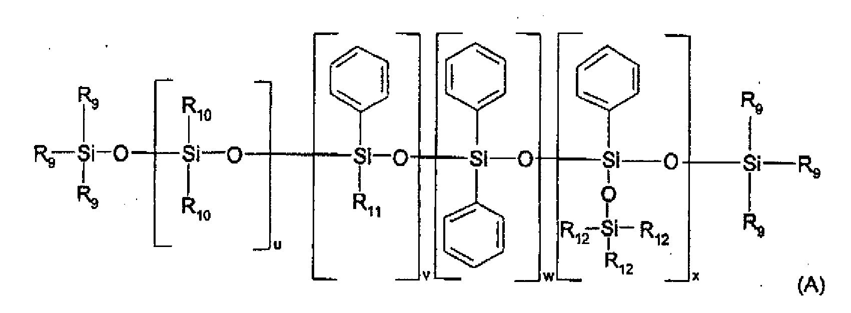 in which:
- R₉ and R₁₂ are each independently a C₁-C₃₀ alkyl radical, an aryl radical or an aralkyl radical,
- R₁₀ and R₁₁ are each independently a C₁-C₃₀ alkyl radical or an aralkyl radical,
- u, v, w and x are each independently integers from 0 to 900,
with the provisos that the sum v+w+x is other than 0 and that the sum u+v+w+x is from 1 to 900; in particular, u+v+w+x is from 1 to 800 and c) a particulate phase, **characterized in that** it contains less than 5% of a volatile oil.

2. Composition according to the preceding claim, **characterized in that** the non-volatile hydrocarbon oil is diisostearyl malate.

3. Composition according to either of the preceding claims, **characterized in that** the non-volatile hydrocarbon oil represents from 5 to 99%, preferably from 10 to 60% and more preferably from 15 to 50% of the total weight of the composition.

4. Composition according to one of the preceding claims, **characterized in that** the high-viscosity phenylsilicone oil has a viscosity at 25°C of from 6 x 10⁻⁴ to 5 x 10⁻³ m²/s (600 to 5 000 cSt).

5. Composition according to one of the preceding claims, **characterized in that** the high-viscosity phenylsilicone oil has a viscosity at 25°C of from 6 x 10⁻⁴ to 3 x 10⁻³ m²/s (600 to 3 000 cSt).

6. Composition according to one of the preceding claims, **characterized in that** the high-viscosity phenylsilicone oil represents from 5 to 99% by weight of the composition.

7. Composition according to one of the preceding claims, **characterized in that** it further comprises a phenylsilicone oil with a low viscosity at 25°C of less than 5 x 10⁻⁴ m²/s (500 cSt).

8. Composition according to the preceding claim, **characterized in that** the low-viscosity phenylsilicone oil has a viscosity at 25°C of from 5 x 10⁻⁶ to 5 x 10⁻⁴ m²/s (5 to 500 cSt).

9. Composition according to Claim 7 or 8, **characterized in that** the low-viscosity phenylsilicone oil has a viscosity at 25°C of from 5 x 10⁻⁶ to 3 x 10⁻⁴ m²/s (5 to 300 cSt).

10. Composition according to any one of Claims 7 to 9, **characterized in that** the low-viscosity phenylsilicone oil has a viscosity at 25°C of from 5 x 10⁻⁶ to 1 x 10⁻⁴ m²/s (5 to 100 cSt).

11. Composition according to either of Claims 7 and 8, **characterized in that** the low-viscosity phenylsilicone oil is selected from the oils of formula (A) as defined in Claim 1.

12. Composition according to one of Claims 7 to 11, **characterized in that** the low-viscosity phenylsilicone oil represents from 5 to 99% by weight of the composition.

13. Composition according to one of Claims 7 to 12, **characterized in that** the ratio by weight between the low-viscosity phenylsilicone oil and the high-viscosity silicone oil is from 1/10 to 10/1.

14. Composition according to one of Claims 7 to 13, **characterized in that** the ratio by weight between the low-viscosity phenylsilicone oil and the high-viscosity silicone oil is from 2/10 to 10/2.

15. Composition according to one of Claims 7 to 14, **characterized in that** the ratio by weight between the low-viscosity phenylsilicone oil and the high-viscosity silicone oil is from 3/10 to 10/5.

16. Composition according to one of the preceding claims, **characterized in that** it further comprises a rheological agent selected from waxes, fatty compounds which are pastelike at ambient temperature of 25°C, lipophilic gelling agents and mixtures thereof.

17. Composition according to the preceding claim, **characterized in that** the rheological agent represents from 0.1 to 65%, preferably from 1 to 50%, more preferably from 3 to 40% and very preferably from 5 to 30% of the total weight of the composition.

18. Composition according to Claim 16 or 17, **characterized in that** the rheological agent is a silicone wax selected from alkyldimethicones or alkoxydimethicones having an alkyl or alkoxy chain of 10 to 45 carbon atoms, poly(di)methylsiloxane esters which are solid at 30°C and whose ester chain contains at least 10 carbon atoms, and mixtures thereof.

19. Composition according to one of the preceding claims, **characterized in that** the rheological agent is an apolar hydrocarbon wax selected from paraffin, lignite wax or microcrystalline wax, ceresin or ozokerite, synthetic waxes such as the polyethylene waxes obtained from the polymerization or copolymerization of ethylene, Fischer-Tropsch waxes, or else di(1,1,1-trimethylolpropane) tetrastearate, and mixtures thereof.

20. Composition according to Claim 16, **characterized in that** the lipophilic gelling agent is an organogeller.

21. Composition according to the preceding claim, **characterized in that** the organogeller is selected from amides of tricarboxylic acid, diamides having hydrocarbon chains each containing 1 to 22 carbon atoms, the said chains being unsubstituted or substituted with at least one substituent selected from ester, urea and fluoro groups, the amides of N-acylamino acids, and mixtures thereof.

22. Composition according to either of Claims 20 and 21, **characterized in that** the organogeller is selected from cyclohexanetricarboxamides, diamides resulting from the reaction of diaminocyclohexane and an acid chloride, diamides resulting from the reaction of an N-acylamino acid with amines containing 1 to 22 carbon atoms, and mixtures thereof.

23. Composition according to one of Claims 16 and 20 to 22, **characterized in that** the lipophilic gelling agent represents from 0.1 to 50%, more preferably 1 to 30% and very preferably from 2 to 20% by weight relative to the total weight of the composition.

24. Composition according to one of the preceding claims, **characterized in that** the particulate phase comprises pigments and/or nacres and/or fillers.

25. Composition according to one of the preceding claims, **characterized in that** the particulate phase is present in a proportion of from 0.01 to 60%, preferably 5 to 25% by weight relative to the total weight of the composition.

26. Composition according to one of the preceding claims, **characterized in that** it is in the form of an exoskeletal appendage and/or lip, body or face care and/or makeup product.

27. Composition according to one of the preceding claims, **characterized in that** it is in the form of a lip makeup product.

28. Composition according to the preceding claim, **characterized in that** it is in anhydrous form.

29. Use of the combination of at least one phenylsilicone oil with a high viscosity of greater than or equal to 5 x 10⁻⁴ m²/s (500 cSt), at least one non-volatile hydrocarbon oil having a molecular mass of more than 600 g/mol which is soluble or dispersible in the said silicone oil, the non-volatile hydrocarbon oil being selected from hydroxyl-containing polyesters, the said hydroxyl-containing polyesters being polyesters of fatty monoalcohol and hydroxyl-containing polycarboxylic acid, in a composition comprising a physiologically acceptable medium and a particulate phase and containing less than 5% of a volatile oil, the said composition having properties of staying power and of gloss.

30. Cosmetic method of imparting properties of staying power and gloss to a film of cosmetic composition comprising a particulate phase and containing less than 5% of a volatile oil, which comprises introducing into the said composition an effective amount of at least one phenylsilicone oil with a high-viscosity of greater than or equal to 5 x 10⁻⁴ m²/s (500 cSt), at least one non-volatile hydrocarbon oil having a molecular mass of more than 600 g/mol which is soluble or dispersible in the said silicone oil, the non-volatile hydrocarbon oil being selected from hydroxyl-containing polyesters, the said hydroxyl-containing polyesters being polyesters of fatty monoalcohol and hydroxyl-containing polycarboxylic acid.

## Patentansprüche

1. Kosmetische Zusammensetzung, umfassend, in einem physiologisch verträglichen Medium, a) mindestens ein hochviskoses Phenylsilikonöl, das eine Viskosität bei 25 °C von 5.10⁻⁴ bis 1.10⁻² m²/s (500 bis 10000 cSt) aufweist, b) mindestens ein nichtflüchtiges Kohlenwasserstofföl mit einer Molmasse von größer als 600 g/mol, ausgewählt aus hydroxylierten Polyestern, wobei die hydroxylierten Polyester Polyester von Fettmonoalkohol und hydroxylierter Polycarbonsäure sind, wobei das hochviskose Phenylsilikonöl aus den Ölen der folgenden Formel (A) ausgewählt ist: 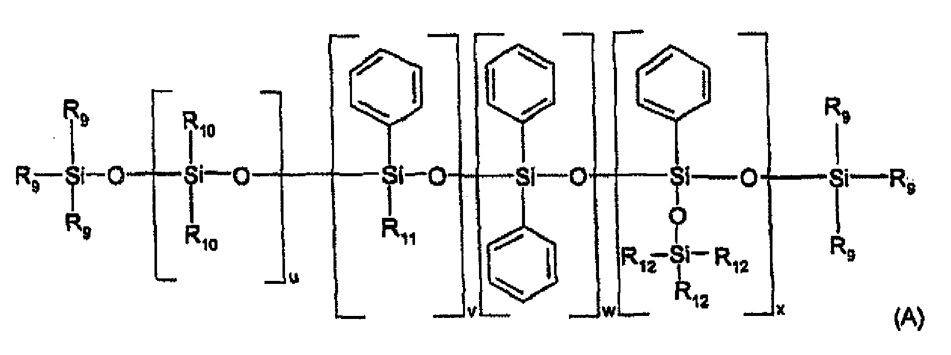 wobei:
- R₉ und R₁₂ jeweils unabhängig einen C₁-C₃₀-Alkylrest, einen Arylrest oder einen Aralkylrest darstellen,
- R₁₀ und R₁₁ jeweils unabhängig einen C₁-C₃₀-Alkylrest oder einen Aralkylrest darstellen,
- u, v, w und x jeweils unabhängig .ganze Zahlen von 0 bis 900 sind, mit der Maßgabe, dass die Summe v+w+x von Null verschieden ist und dass die Summe u+v+w+x von 1 bis 900 reicht, insbesondere dass u+v+w+x von 1 bis 800 reicht, und c) eine Teilchenphase, **dadurch gekennzeichnet, dass** sie weniger als 5 % eines flüchtigen Öls umfasst.

2. Zusammensetzung nach einem der vorhergehenden Ansprüche, **dadurch gekennzeichnet, dass** das nichtflüchtige Kohlenwasserstofföl Diisostearylmalat ist.

3. Zusammensetzung nach einem der vorhergehenden Ansprüche, **dadurch gekennzeichnet, dass** das nichtflüchtige Kohlenwasserstofföl 5 bis 99 %, vorzugsweise 10 bis 60 % und besser 15 bis 50 % des Gesamtgewichts der Zusammensetzung darstellt.

4. Zusammensetzung nach einem der vorhergehenden Ansprüche, **dadurch gekennzeichnet, dass** das hochviskose Phenylsilikonöl eine Viskosität bei 25 °C von 6.10⁻⁴ bis 5.10⁻³ m²/s (600 bis 5000 cSt) aufweist.

5. Zusammensetzung nach einem der vorhergehenden Ansprüche, **dadurch gekennzeichnet, dass** das hochviskose Phenylsilikonöl eine Viskosität bei 25 °C von 6.10⁻⁴ bis 3.10⁻³ m²/s (600 bis 3000 cSt) aufweist.

6. Zusammensetzung nach einem der vorhergehenden Ansprüche, **dadurch gekennzeichnet, dass** das hochviskose Phenylsilikonöl 5 bis 99 Gew.-% der Zusammensetzung darstellt.

7. Zusammensetzung nach einem der vorhergehenden Ansprüche, **dadurch gekennzeichnet, dass** sie weiterhin ein Phenylsilikonöl von niedriger Viskosität bei 25 °C von kleiner als 5.10⁻⁴ (500 cSt) enthält.

8. Zusammensetzung nach einem der vorhergehenden Ansprüche, **dadurch gekennzeichnet, dass** das niederviskose Phenylsilikonöl eine Viskosität bei 25 °C von 5.10⁻⁶ bis 5.10⁻⁴ m²/s (5 bis 500 cSt) aufweist.

9. Zusammensetzung nach Anspruch 7 oder 8, **dadurch gekennzeichnet, dass** das niederviskose Phenylsilikonöl eine Viskosität bei 25 °C von 5.10⁻⁶ bis 3.10⁻⁴ m²/s (5 bis 300 cSt) bei 25 °C aufweist.

10. Zusammensetzung nach einem der Ansprüche 7 bis 9, **dadurch gekennzeichnet, dass** das niederviskose Phenylsilikonöl eine Viskosität bei 25 °C von 5.10⁻⁶ bis 1.10⁻⁴ m²/s (5 bis 100 cSt) bei 25 °C aufweist.

11. Zusammensetzung nach einem der Ansprüche 7 oder 8, **dadurch gekennzeichnet, dass** das niederviskose Phenylsilikonöl aus den Ölen der Formel (A), wie in Anspruch 1 definiert, ausgewählt ist.

12. Zusammensetzung nach einem der Ansprüche 7 bis 11, **dadurch gekennzeichnet, dass** das niederviskose Phenylsilikonöl 5 bis 99 Gew.-% der Zusammensetzung darstellt.

13. Zusammensetzung nach einem der Ansprüche 7 bis 12, **dadurch gekennzeichnet, dass** das Gewichtsverhältnis zwischen niederviskosem Phenylsilikonöl und hochviskosem Phenylsilikonöl von 1/10 bis 10/1 reicht.

14. Zusammensetzung nach einem der Ansprüche 7 bis 13, **dadurch gekennzeichnet, dass** das Gewichtsverhältnis zwischen niederviskosem Phenylsilikonöl und hochviskosem Phenylsilikonöl von 2/10 bis 10/2 reicht.

15. Zusammensetzung nach einem der Ansprüche 7 bis 14, **dadurch gekennzeichnet, dass** das Gewichtsverhältnis zwischen niederviskosem Phenylsilikonöl und hochviskosem Phenylsilikonöl von 3/10 bis 10/5 reicht.

16. Zusammensetzung nach einem der vorhergehenden Ansprüche, **dadurch gekennzeichnet, dass** sie weiterhin ein rheologisches Mittel umfasst, das ausgewählt ist aus Wachsen, bei Umgebungstemperatur von 25 °C pastösen Fettverbindungen, lipophilen Gelbildnem und ihren Gemischen.

17. Zusammensetzung nach dem vorhergehenden Anspruch, **dadurch gekennzeichnet, dass** das rheologische Mittel 0,1 bis 65 %, vorzugsweise 1 bis 50 %, besser 3 bis 40 % und noch besser 5 bis 30 Gew.-% des Gesamtgewichts der Zusammensetzung darstellt.

18. Zusammensetzung nach Anspruch 16 oder 17, **dadurch gekennzeichnet, dass** das rheologische Mittel ein Silikonwachs ist, das ausgewählt ist aus Alkyldimethiconen oder Alkoxydimethiconen mit einer Alkyl- oder Alkoxykette von 10 bis 45 Kohlenstoffatomen, bei 30 °C festen Poly(di)methylsiloxanestern, deren Esterkette mindestens 10 Kohlenstoffatome umfasst, und ihren Gemischen.

19. Zusammensetzung nach einem der vorhergehenden Ansprüche, **dadurch gekennzeichnet, dass** das rheologische Mittel ein apolares Kohlenwasserstoffwachs ist, das ausgewählt ist aus Paraffin, Lignit- oder mikrokristallinen Harzen, Ceresin oder Ozokerit, synthetischen Wachsen, wie Polyethylenwachsen, die aus der Polymerisation oder Copolymerisation von Ethylen hervorgehen, Fischer-Tropsch-Wachsen oder Di-(trimethylol-1,1,1-propan)-tetrastearat und ihren Gemischen.

20. Zusammensetzung nach Anspruch 16, **dadurch gekennzeichnet, dass** der lipophile Gelbildner ein organischer Gelbildner ist.

21. Zusammensetzung nach einem der vorhergehenden Ansprüche, **dadurch gekennzeichnet, dass** der organische Gelbildner ausgewählt ist aus Amiden von Tricarbonsäuren, Diamiden mit Kohlenwasserstoffketten, die jeweils 1 bis 22 Kohlenstoffatome enthalten, wobei die Ketten nicht substituiert sind oder mit mindestens einem Substituenten substituiert sind, ausgewählt aus Ester-, Harnstoff oder Flourgruppen, Amiden von N-Acylaminosäuren und ihren Gemischen.

22. Zusammensetzung nach einem der Ansprüche 20 oder 21, **dadurch gekennzeichnet, dass** der organische Gelbildner ausgewählt ist aus Cycolhexantricarboxamiden, Diamiden, die aus der Reaktion des Diaminocyclohexans und einem Säurechlorid hervorgehen, Diamiden, die aus der Einwirkung einer N-Acylaminosäure auf Amine, die 1 bis 22 Kohlenstoffatome einschießen, hervorgehen, und ihren Gemischen.

23. Zusammensetzung nach einem der Ansprüche 16 und 20 bis 22, **dadurch gekennzeichnet, dass** der lipophile Gelbildner 0,1 bis 50 Gew.-%, besser 1 bis 30 % und noch besser 2 bis 20 %, bezogen auf das Gesamtgewicht der Zusammensetzung, darstellt.

24. Zusammensetzung nach einem der vorhergehenden Ansprüche, **dadurch gekennzeichnet, dass** die Teilchenphase Pigmente und/oder Lacke und/oder Füllstoffe umfasst.

25. Zusammensetzung nach einem der vorhergehenden Ansprüche, **dadurch gekennzeichnet, dass** die Teilchenphase in einem Verhältnis von 0,01 bis 60 Gew.-%, vorzugsweise von 5 bis 25 Gew.-%, bezogen auf das Gesamtgewicht der Zusammensetzung, vorhanden ist.

26. Zusammensetzung nach einem der vorhergehenden Ansprüche, **dadurch gekennzeichnet, dass** sie in der Form eines Schrninkprodukts und/oder Gesichts- oder Körperpflege-, Lippen- und/oder Phanerenpflegeprodukts vorliegt.

27. Zusammensetzung nach einem der vorhergehenden Ansprüche, **dadurch gekennzeichnet, dass** sie in der Form eines Lippenschminkprodukts vorliegt.

28. Zusammensetzung nach einem der vorhergehenden Ansprüche, **dadurch gekennzeichnet, dass** sie in wasserfreier Form vorliegt.

29. Verwendung der Kombination von mindestens einem Phenylsilikonöl von hoher Viskosität von größer oder gleich 5.10⁻⁴ m²/s (500 cSt), von mindestens einem nichtflüchtigen Kohlenwasserstofföl einer Molmasse von größer als 600 g/mol, das in dem Silikonöl löslich oder dispergierbar ist, wobei das nichtflüchtige Kohlenwasserstofföl aus hydroxylierten Polyestern ausgewählt ist, wobei die hydroxylierten Polyester Polyester von Fettmonoalkohol und hydroxylierter Polycarbonsäure sind, in einer Zusammensetzung, die ein physiologisch verträgliches Medium und eine Teilchenphase umfasst und weniger als 5 % eines flüchtigen Öls umfasst, wobei die Zusammensetzung Eigenschaften von Festigkeit und Glanz aufweist.

30. Kosmetisches Verfahren zum Verleihen eines Films von kosmetischer Zusammensetzung, umfassend eine Teilchenphase und weniger als 5 % von einem flüchtigen Öl, mit den Eigenschaften von Festigkeit und Glanz, bestehend aus dem Einbringen in die Zusammensetzung einer wirksamen Menge von mindestens einem Phenylsilikonöl von hoher Viskosität von größer oder gleich 5.10⁻⁴ m²/s (500 cSt), mindestens einem nichtflüchtigen Kohlenwasserstofföl mit einer Molmasse von größer als 600 g/mol, das in dem Silikonöl löslich oder dispergierbar ist, wobei das nicht flüchtige Kohlenwasserstofföl ausgewählt ist aus hydroxylierten Polyestern, wobei die hydroxylierten Polyester Polyester von Fettmonoalkohol und hydroxylierter Polycarbonsäure sind.
